# EUROPÄISCHE PATENTSCHRIFT

(11) **EP 0 553 070 B1**
(45) Veröffentlichungstag und Bekanntmachung des Hinweises auf die Patenterteilung: **23.10.1996**
(21) Anmeldenummer: 93890007.3
(22) Anmeldetag: 18.01.1993
(51) Int. Cl.: C07C 291/04, C08B 1/00, G01N 21/71, G01J 3/36

(54) **Aminoxide**
Amine oxides
Amines oxides

(30) Priorität: 23.01.1992 AT 109/92
(43) Veröffentlichungstag der Anmeldung: 28.07.1993
(73) Patentinhaber: Lenzing Aktiengesellschaft, A-4860 Lenzing (AT)
(72) Erfinder: Astegger, Stephan, Dr., A-4850 Timelkam (AT); Eichinger, Dieter, Dr., A-4840 Vöcklabruck (AT); Falk, Heinz, Prof. Dr., A-4020 Linz (AT); Teubl, Günter, A-4861 Schörfling (AT)
(74) Vertreter: Schwarz, Albin, Dr. Kopecky & Schwarz Patentanwälte

(56) Entgegenhaltungen:
- EP-A- 0 393 908
- DE-A- 2 848 471
- DE-A- 2 848 471
- US-A- 3 449 431
- US-A- 3 449 432
- PATENT ABSTRACTS OF JAPAN, vol. 9, no. 17, (C-262)(1740), 24. Januar 1985; & JP-A-59 167 558

## Beschreibung

Die Erfindung betrifft tertiäre Aminoxide, welche zur Herstellung form- bzw. spinnbarer cellulosischer Lösungen verwendet werden können.

Aus der US-PS 2,179,181 ist bekannt, daß tertiäre Aminoxide Cellulose ohne Derivatisierung zu lösen vermögen und daß aus diesen Lösungen durch Fällung cellulosische Formkörper wie Fasern gewonnen werden können. In den US-PSen 3,447,939, 3,447,956 und 3,508,941 werden weitere Verfahren zur Herstellung cellulosischer Lösungen beschrieben, wobei als Lösungsmittel bevorzugt cyclische Aminoxide eingesetzt werden.

Die genannten Verfahren weisen jedoch gemäß US-A-4,196,282 den Nachteil auf, daß sie nur die Herstellung relativ verdünnter Lösungen mit einem Cellulose-Gehalt von maximal 10 Masse-% ermöglichen. Gemäß der zitierten US-A-4,196,282 sind höhere Cellulose-Konzentrationen nur dann erreichbar, wenn wasserhältiges Aminoxid eingesetzt wird bzw. wenn zusätzlich Wasser zur Aminoxid/Cellulose-Mischung zugegeben wird. Es wird vorgeschlagen, in der Mischung bis 29 Masse-% Wasser vorzusehen. Nachteilig dabei ist aber, daß die aminoxidhältigen Volumina, die nach der Fällung anfallen und aufgearbeitet werden müssen, sehr groß werden.

Beim heutzutage meistverwendeten Lösungsmittel, N-Methylmorpholin-N-oxid (NMMO), muß Wasser oder ein anderes mit NMMO mischbares Nichtlösungsmittel der Cellulose-NMMO-Suspension zugesetzt werden, da NMMO bei Raumtemperatur im festen Zustand vorliegt. Dazu kommt noch, daß die erhaltene Celluloselösung eine hohe Kristallisationsneigung besitzt, was ebenfalls nachteilig ist.

Die Erfindung bezweckt die Vermeidung dieser Nachteile und stellt sich zur Aufgabe, tertiäre Aminoxide zur Verfügung zu stellen, welche gestatten, form- bzw. spinnbare Celluloselösungen mit möglichst wenig Neigung zur Kristallisation herzustellen. Eine weitere Aufgabe besteht darin, Celluloselösungen mit einem möglichst kleinen Wasseranteil und mit einem Cellulose-Gehalt von mehr als 10 Masse-%, bezogen auf die Masse der Lösung, zur Verzügung zu stellen.

Die erfindungsgemäß verwendeten tertiären Aminoxide besitzen die allgemeine Formel 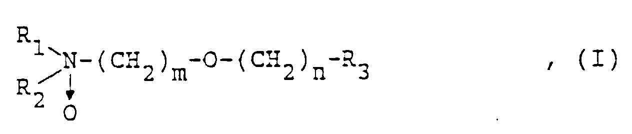 worin R₁ und R₂ Alkylgruppen mit 1-4 C-Atomen darstellen, R₃ für Wasserstoff oder eine Hydroxylgruppe, m für 2 oder 3 und n für 1 oder 2 steht.

Insbesondere gut geeignet haben Sich Aminoxide der obigen allgemeinen Formel (I) erwiesen, wenn m die Zahl 2 und n eine der Zahlen 1 oder 2 bedeuten.

Als ausgezeichnetes Lösungsmittel hat sich das 2-(N,N-Dimethylaminoethoxy)-ethanol-N-oxid der Formel 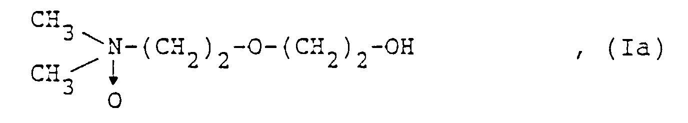 erwiesen. Dies ist insofern überraschend, da in der US-A-4,196,282 eine strukturell sehr ähnliche Verbindung als schlechtes Lösungsmittel für Cellulose beschrieben wird. Bei dieser Verbindung handelt es sich um das 2-(2-Hydroxypropoxy)-N-ethyl-N,N-dimethylamin-N-oxid, welches nur bis zu 7,5 Masse-% (bezogen auf die Lösung) Cellulose aufnehmen kann und auch das nur dann, wenn zwischen 5 und 10 Masse-% Wasser in der Lösung anwesend sind (siehe Tabelle in Spalte 6 der US-A-4,196,282). Demgegenüber gestatten die erfindungsgemäß verwendeten Aminoxide die Herstellung von praktisch wasserfreien Celluloselösungen mit einem Cellulose-Gehalt von über 10 Masse-% (bezogen auf die Lösung). Natürlich kann die erfindungsgemäße Lösung Wasser oder ein anderes Nichtlösungsmittel (für Cellulose) als Hilfsstoff enthalten.

Die erfindungsgemäßen Verbindungen können durch Oxydation der entsprechenden an sich bekannten tertiären Amine der allgemeinen Formel 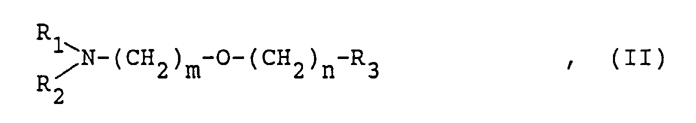 worin R₁, R₂, R₃, m und n obige Bedeutung haben, hergestellt werden. Die Überführung tertiärer Amine in ihre N-Oxide ist allgemein bekannt und kann z.B. durch Behandeln mit H₂O₂ durchgeführt werden.

Die erfindungsgemäßen Aminoxide sind wesentlich billiger herstellbar als das heute vielfach verwendete N-Methylmorpholin-N-Oxid (NMMO), da die entsprechenden Ausgangsamine in größerem Maßstab hergestellt werden, als Morpholin bzw. Methylmorpholin. Darüberhinaus sind die erfindungsgemäß verwendeten Aminoxide wesentlich leichter biologisch abbaubar (bestimmt nach dem Zahn-Welles-Test), als cyclische Aminoxide.

Es hat sich gezeigt, daß die erfindungsgemäß verwendeten Aminoxide Cellulose leicht zu lösen vermögen. Die erfindungsgemäßen Celluloselösungen besitzen eine wesentlich geringere Kristallisationsneigung als NMMO-Lösungen. Eine bevorzugte Ausführungsform der erfindungsgemäßen form- bzw. spinnbaren Celluloselösung ist wasserfrei und enthält über 10 Masse-% Cellulose.

Es hat sich gezeigt, daß auch Mischungen der erfindungsgemäß verwendeten Aminoxide mit NMMO zur Herstellung von Celluloselösungen verwendet werden können, die sich selbst bei Raumtemperatur noch plastisch verformen lassen, sofern der Anteil des NMMO unter 70 Masse-% (bezogen auf das Aminoxidgemisch) liegt.

Die erfindungsgemäße Lösung kann noch zusätzlich einen Stabilisator enthalten. Als besonders geeignet erwiesen haben sich Verbindungen der Flavongruppe, wie Rutin (3,3',4',5,7-Pentahydroxyflavon-3-rutinocid), Quercetin (3',3,3',5,7-Pentahydroxyflavon), oder Morin (2',3,4',5,7-Pentahydroxyflavon), vorzugsweise in Mengen von 0,001 bis 1,5 Masse-%, bezogen auf die Form- bzw. Spinnmasse. Diese Stabilisatoren sind aus der AT-B 393 841 bekannt. Die in der EP-A-0 111 518, der EP-A-0 047 929, der DD-A-218 104 und der DD-A-229 708 beschriebenen Stabilisatoren können ebenfalls mit Erfolg eingesetzt werden.

Als Stabilisator kann auch ein Gemisch aus H₂O₂ und Oxalsäure eingesetzt werden. Es hat sich gezeigt, daß die bei der Herstellung der Lösung und beim Erwärmen üblicherweise auftretende Verfärbung vermindert wird, wenn in der Lösung 0,01 bis 1 %, vorzugsweise 0,1 %, H₂O₂ und 0,03 bis 2 %, vorzugsweise 0,1 %, eines Stabilisators für H₂O₂, vorzugsweise Oxalsäure, vorgesehen werden.

Die erfindungsgemäße Lösung kann hergestellt werden, indem ein erfindungsgemäß verwendetes Aminoxid, gegebenenfalls zusammen mit NMMO, mit zerkleinerter Cellulose und gegebenenfalls einem weiteren löslichen Polymeren vermischt und erwärmt wird, wobei eventuell vorhandenes Wasser gegebenenfalls abgezogen wird. Als weitere Polymere eignen sich z.B. Polyamide, Celluloseacetat oder Polyester.

Die erfindungsgemäß verwendeten Aminoxide können sogar mit einem Wassergehalt von etwa 50 Masse-% zur Herstellung der Celluloselösungen eingesetzt werden. In diesem Fall hat sich das in der EP-A-0 356 419 beschriebene Verfahren zur Bereitung der Celluloselösung besonders bewährt. Bei geringen Wassergehalten oder bei Wasserfreiheit ist es möglich, die Celluloselösung in einem Extruder, einem Barrelmischer oder ähnlichem, einem Rührkessel oder einem Tellermischer herzustellen.

Cellulosische Formkörper werden bevorzugt so hergestellt, daß die erfindungsgemäße form- bzw. spinnbare Lösung durch eine formgebende Vorrichtung gepreßt und gegebenenfalls verstreckt und koaguliert wird. Es hat sich gezeigt, daß die erfindungsgemäße Lösung nach jedem bekannten Verfahren, wie Naßspinnen und Trocken/Naßspinnen ("Luftspaltspinnen") verarbeitet und zur Ausbildung von Filmen eingesetzt werden kann.

Aus dem Fällbad können die gelösten erfindungsgemäßen Aminoxide auf einfache Weise abgetrennt und wiedergewonnen werden, indem die zu reinigenden Lösungen mit einem Anionenaustauscher in Kontakt gebracht und die gereinigten Lösungen vom Anionenaustauscher abgetrennt werden, wobei die Reinigung in einem einstufigen Verfahren mit einem Anionenaustauscher durchgeführt wird, der als funktionelle Gruppe ausschließlich quartäre Tetraalkylammoniumgruppen der Formeln

-CH₂-N⁺(CH₃)₃X⁻ oder -CH₂-N⁺[(CH₃)₂(CH₂OH)]X⁻

aufweist, worin X⁻ das Anion einer anorganischen oder organischen Säure darstellt, worauf der Anionenaustauscher mit einer wässerigen sauren Lösung regeneriert wird. Vorteilhaft wird ein Anionenaustauscher eingesetzt, dessen Anion X⁻ von einer flüchtigen Säure, insbesondere von Kohlensäure, Ameisensäure oder Essigsäure, abgeleitet ist. Die Regenerierung des Anionenaustauschers kann mit einer wässerigen Lösung von Ameisensäure, Essigsäure oder Kohlensäure durchgeführt werden, welche Lösung weiters bis zu 5 Masse-% einer Hydroxycarbonsäure, insbesondere Weinsäure, enthält. Ein derartiges Reinigungsverfahren von Spinnbadlösungen ist zur Abtrennung von NMMO aus der EP-A-0 427 701 bekannt.

Die erfindungsgemäß verwendeten Aminoxide können aber auch nach dem in der EP-A-0 402 347 beschriebenen Verfahren abgetrennt werden. Gemäß diesem Verfahren wird das gebrauchte Fällbad mit einem Kationenaustauscherharz in Kontakt gebracht, um den Kationenaustauscher mit den Aminoxiden zu beladen, worauf der beladene Kationenaustauscher gewaschen und die Aminoxide eluiert werden, wobei ein Kationenaustauscherharz eingesetzt wird, dessen Ankergruppen aus Carboxylgruppen bestehen und wobei der mit Aminoxiden beladene Kationenaustauscher mit einer wässerigen Lösung einer schwachen Säure mit einem pKₐ-Wert von größer als 3,0 behandelt wird, um die Aminoxide zu eluieren.

Zur Reinigung von wässerigen Aminoxid-Lösungen, insbesondere Spinnbadlösungen, können die Lösungen auch mit Adsorptionsmitteln in Kontakt gebracht und anschließend einer Filtration unterworfen werden. Als Adsorptionsmittel wird vorzugsweise Aluminiumoxid, Siliziumoxid oder Kohle eingesetzt. Die Adsorptionsmittel weisen zweckmäßigerweise eine Korngröße < 0,15 mm auf. Die Anwesenheit der Adsorptionsmittel erleichtert die Filtration eventuell vorhandener Trübstoffe.

Es ist weiters möglich, zur Gewinnung einer aufkonzentrierten Lösung der erfindungsgemäßen Aminoxide bzw. deren Amine aus der gebrauchten Spinnbadlösung Wasser abzutrennen, indem die Spinnbadlösung in einer Umkehrosmoseanlage mit einem Druck, der größer ist als der osmotische Druck, durch eine semipermeable Membran gepreßt wird. Ein derartiges Verfahren wird für NMMO-Lösungen in der EP-A-0 448 924 beschrieben.

Die Erfindung wird mit den nachfolgenden Beispielen noch näher erläutert, wobei sich das Beispiel 1 auf die Synthese des 2-(N,N-Dimethylaminoethoxy)-ethanol-N-oxids und anderer tertiärer Aminoxide bezieht und die Beispiele 2 bis 10 die Herstellung cellulosischer Lösungen und zum Teil deren Weiterverarbeitung betreffen.

Mit sämtlichen der in Beispiel 1 angeführten N-Oxide konnten Celluloselösungen mit einem Cellulosegehalt von über 10 Masse-% (bezogen auf die Lösung) hergestellt werden, die praktisch wasserfrei waren und eine äußerst geringe Kristallisationsneigung besaßen.

### Beispiel 1:

Zu 788 g (= 5,9 Mol) 2-(N,N-Dimethylaminoethoxy)-ethanol werden insgesamt 802 g wässeriges H₂O₂ (30 %-ig; Gehaltsbestimmung durch Redoxtitration mit KMnO₄) enthaltend 240,6 (= 7,08 Mol) H₂O₂ in mehreren Portionen derart zugegeben, daß die Temperatur der Reaktionslösung nicht über 30°C ansteigt; wenn notwendig, wird das Reaktionsgefäß in einem Glykolbad (Temperatur <0°C) gekühlt. Im Laufe der Oxydation werden insgesamt 70 ml Wasser zur Verdünnung zugegeben. Nach Abschluß der H₂O₂-Zugabe läßt man die Reaktionsmischung auf Raumtemperatur erwärmen. Die Reaktionsmischung wird über Nacht gerührt, danach auf ca. 70°C erwärmt und bei dieser Temperatur etwa 3 Stunden belassen. Danach wird eine Katalaselösung (ca. 1 %) zur Zerstörung des überschüssigen H₂O₂ zugegeben, wobei die Zugabe solange wiederholt wird, bis die Schaumentwicklung aufhört. Die derart erhaltene Lösung ist wasserklar und weist eine Aminoxidkonzentration von ca. 50 % auf.

Zur Isolierung des Aminoxides wird aus der erhaltenen Lösung zunächst durch azeotrope Destillation mit Benzol Wasser abgeschieden. Danach trennt man das Aminoxid von der Benzolphase und rotiert die letzten Reste Benzol ab. Es werden Kristalle erhalten, welche mit trockenem Aceton umkristallisiert und im Vakuum getrocknet werden.

Das Aminoxid besitzt nachstehende physikalische Daten:

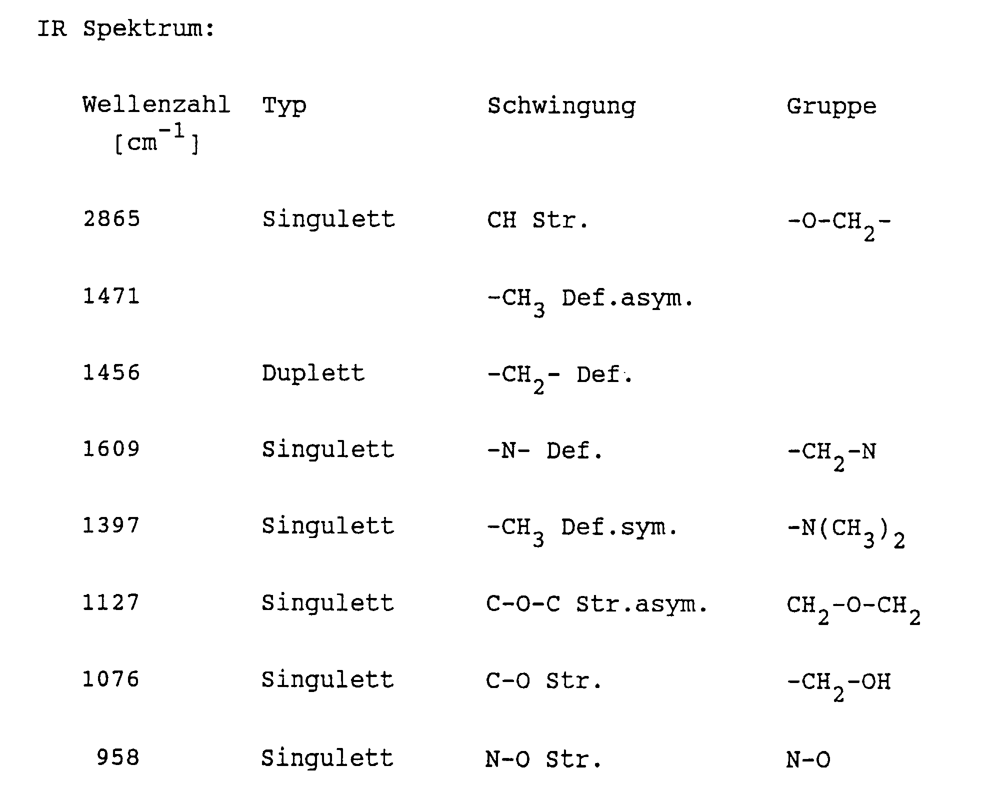

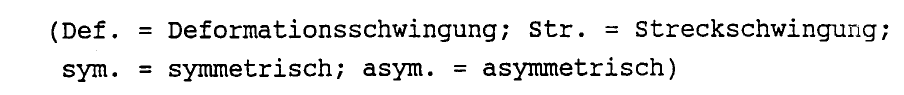

Die 46,9 %-ige und 96 %-ige wässerige Lösung des erhaltenen Aminoxids besitzt einen Brechungsindex (gemessen mit Tageslicht) von 1,4100 bzw. von 1,4830 (bei 20°C), woraus sich für das Aminoxid selbst ein Brechungsindex von 1,4889 errechnet.

Gemäß der obigen Verfahrensvorschrift wurden weitere Aminoxide aus den entsprechenden tertiären Aminen hergestellt und mittels ¹H-NMR-Spektroskopie charakterisiert. Diese Daten sind in der nachfolgenden Tabelle zusammengefaßt.

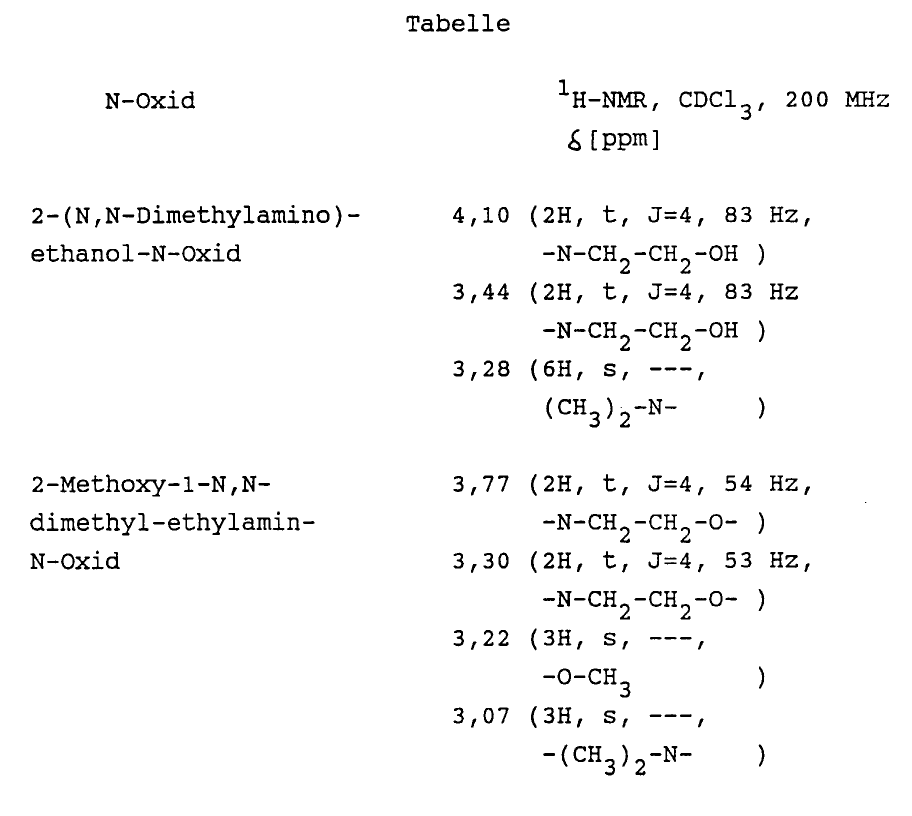

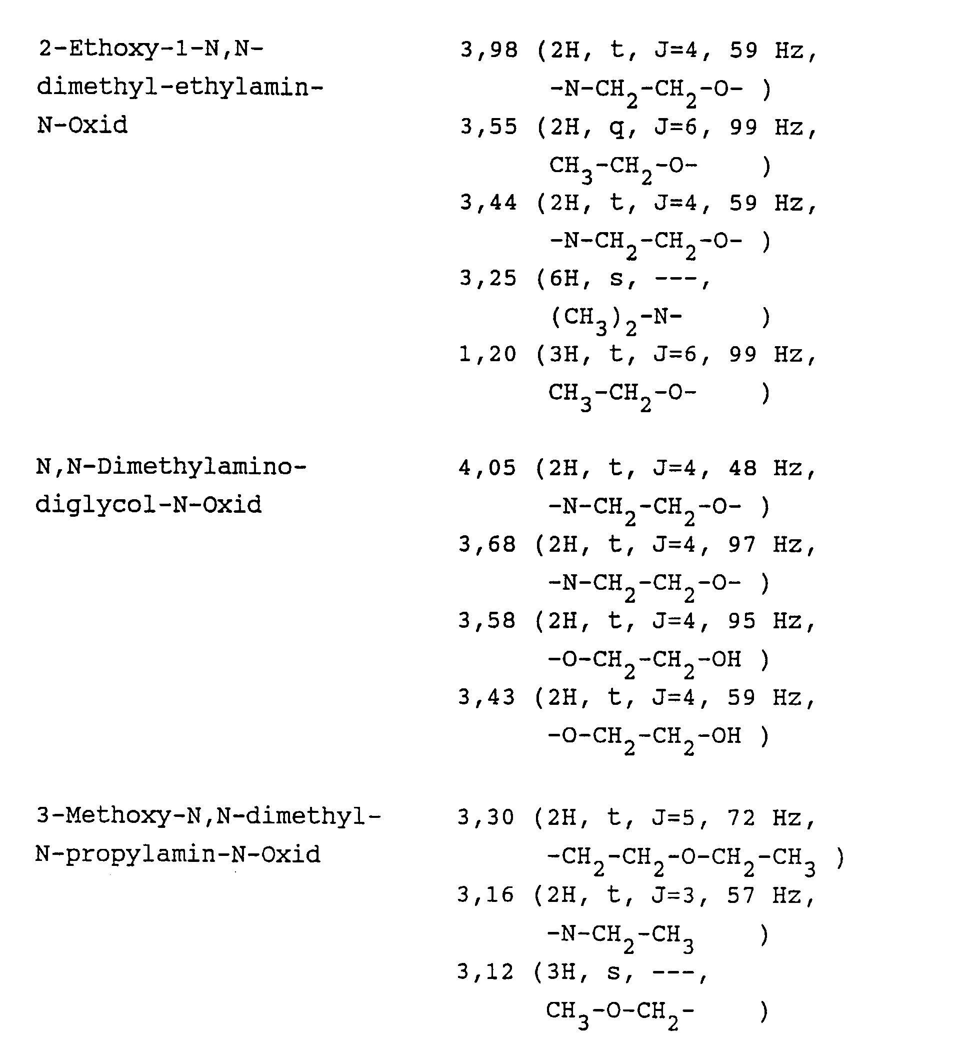

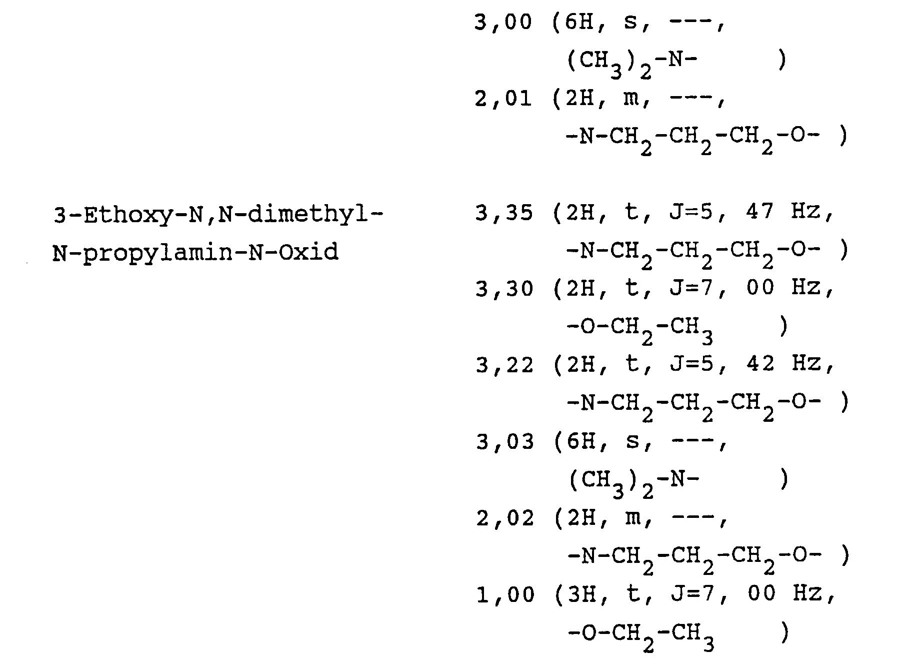

### Beispiel 2:

167 g 2-(N,N-Dimethylaminoethoxy)-ethanol-N-oxid (Gehalt: 96 %) werden mit 43 g Cellulose (Buckeye V5; Feuchte: 7 %) zu einer Suspension vermischt und als Stabilisator 0,08 g Rutin zugesetzt. Danach werden in einem Kneter unter Vakuum 10 g Wasser abdestilliert. Man erhält eine braungefärbte, klare Celluloselösung mit einer Cellulosekonzentration von 20 % und einer Aminoxidkonzentration von 80 %.

### Beispiel 3:

177,1 g 2-(N,N-Dimethylaminoethoxy)-ethanol-N-oxid (Wassergehalt: 4 %) werden mit 37,3 g Cellulose (Buckeye V5; Trockengehalt: 93 %) vermischt und mit 0,08 g Rutin als Stabilisator versetzt. Danach werden in einem Kneter unter Vakuum 9 g Wasser abdestilliert. Man erhält eine braungefärbte, klare Celluloselösung mit einer Cellulosekonzentration von 16,9 % und einer Aminoxidkonzentration von 82,8 %. Der Gehalt an Wasser beträgt 0,4 %.

### Beispiel 4:

135 g 2-(N,N-Dimethylaminoethoxy)-ethanol-N-oxid (Gehalt 96 %) werden in einem Kneter mit 15 g Cellulose (Buckeye V5; Feuchte 7 %) und mit 0,08 g Rutin als Stabilisator vermischt und unter Vakuum 7 g Wasser abgezogen. Man erhält eine braungefärbte, klare Lösung mit einer Cellulosekonzentration von 10 % und einer Aminoxidkonzentration von 90 %. Aus dieser Lösung wurden Cellulose-Fasern mit folgenden Eigenschaften ersponnen:
Titer: 2,72 dtex
Festigkeit kond.: 36 cN/tex
Dehnung kond.: 8,5 %
Schlingenfestigkeit: 17 cN/dtex
Schlingendehnung: 2,7 %

Die in einem Wasserbad ausgefällte Cellulose weist einen Polymerisationsgrad (DP) von 500 auf (Cuen Methode).

### Beispiel 5:

317,7 g 2-(N,N-Dimethylaminoethoxy)-ethanol-N-oxid (Wassergehalt: 53,1 %) werden mit 16,1 g Cellulose (Buckeye V5; Trockengehalt 93 %) vermischt und mit 0,08 g Rutin als Stabilisator versetzt. Danach werden in einem Kneter unter Vakuum 159,7 g Wasser abdestilliert. Man erhält eine braungefärbte, klare Celluloselösung mit 9,4 % Cellulose, 85,6 % Aminoxid und 5,0 % Wasser. Die in Wasser ausgefällte Cellulose weist einen DP von 510 auf.

### Beispiel 6:

18 g Cellulose (Buckeye V5; Trockengehalt 93 %) werden mit 69 g 2-(N,N-Dimethylaminoethoxy)-ethanol-N-oxid (Gehalt 90 %) und 117,2 g NMMO (Gehalt 59,7 %) vermischt und als Stabilisator 0,06 g Rutin zugegeben. In einem Kneter werden 44 g Wasser abdestilliert. Man erhält eine klare, braune Celluloselösung mit 11,7 % Cellulose und 88,8 % N-Oxid. Die in Wasser ausgefällte Cellulose weist einen DP von 570 auf.

### Beispiel 7:

198 g 2-(N,N-Dimethylaminoethoxy)-ethanol-N-oxid, (Wassergehalt 21,2 %) werden mit 25,8 g BKZ (Sulfitzellstoff, Trockengehalt 93 %) vermischt und mit 0,1 g Gallussäurepropylester als Stabilisator versetzt. Danach werden in einem Kneter ca. 15 g Wasser abdestilliert. Nach einer Stunde werden 20 g N-Methylmopholin-N-oxid-monohydrat zugesetzt und weitergeknetet, bis eine klare Lösung erhalten wird. Die Lösung enthält 11 % Cellulose, 80 % Aminoxide und 9,3 % Wasser.

Aus dieser Lösung wurden Cellulose-Fasern mit folgenden Eigenschaften ersponnen:
- Titer: 1,60 dtex
- Festigkeit kond.: 38 cN/tex
- Dehnung kond.: 8,8 %
- Schlingenfestigkeit: 18,2 cN/tex
- Schlingendehnung: 2,4 %

### Beispiel 8:

198 g 2-(N,N-Dimethylaminoethoxy)-ethanol-N-oxid, (Wassergehalt 21,2 %) werden mit 25,8 g BKZ (Sulfitzellstoff, Trockengehalt 93 %) vermischt und mit 0,2 g Oxalsäure/Wasserstoffperoxidgemisch als Stabilisator versetzt. Danach werden in einem Kneter ca. 15 g Wasser abdestilliert. Nach einer Stunde werden 20 g N-Methylmorpholin-N-oxid-monohydrat zugesetzt und weitergeknetet, bis eine klare Lösung entstanden ist.

### Beispiel 9:

196 g 2-(N,N-Dimethylaminoethoxy)-ethanol-N-oxid, (Wassergehalt 21,2 %) werden mit 25,8 g Viscokraft LV (Sulfatzellstoff, Trockengehalt 93 %) sowie 2 g Polyvinylacetat als zusätzliche Polymerkomponente vermischt und mit 0,1 g Hydroxyethyliden-1,1-diphosphonsäure als Stabilisator versetzt. Danach werden in einem Kneter ca. 40 g Wasser abdestilliert. Nach einer Stunde werden 20 g N-Methylmorpholin-N-oxid-monohydrat zugesetzt und weitergeknetet, bis eine klare Lösung erhalten wird. Die Lösung enthält 11,8 % Cellulose, 1 % Polyvinylacetat, 84,6 % Aminoxide und 2,5 % Wasser.

### Beispiel 10:

174 g 2-(N,N-Dimethylaminoethoxy)-ethanol-N-oxid, (Wassergehalt 11,2 %) werden mit 24,0 g Viscokraft LV (Sulfatzellstoff, Trockengehalt 93 %) sowie 2 g Polyvinylalkohol als zusätzliche Polymerkomponente vermischt und mit 0,1 g Rutin als Stabilisator versetzt. Danach werden in einem Kneter ca. 21 g Wasser abdestilliert. Nach einer Stunde werden 20 g N-Methylmorpholin-N-oxid-monohydrat zugesetzt und weitergeknetet, bis eine klare Lösung erhalten wird. Die Lösung enthält 11 % Cellulose, 1 % Polyvinylalkohol, 84,3 % Aminoxide und 3,7 % Wasser.

Aus dieser Lösung wurden Cellulose-Fasern mit folgenden Eigenschaften ersponnen:
- Titer: 1,36 dtex
- Festigkeit kond.: 28,8 cN/tex
- Dehnung kond.: 10,2 %
- Schlingenfestigkeit: 12,8 cN/tex
- Schlingendehnung: 2,2 %

## Patentansprüche

1. Aminoxide der allgemeinen Formel 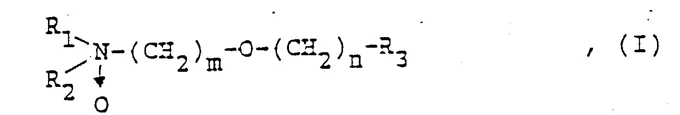 worin R₁ und R₂ Alkylgruppen mit 1-4 C-atomen darstellen, R₃ für Wasserstoff oder eine Hydroxylgruppe, m für 2 oder 3 und n für 1 oder 2 steht.

2. Aminoxide der allgemeinen Formel (I) nach Anspruch 1, worin m die Zahl 2 und n eine der Zahlen 1 oder 2 bedeuten.

3. 2-(N,N-Dimethylaminoethoxy)-ethanol-N-oxid der Formel

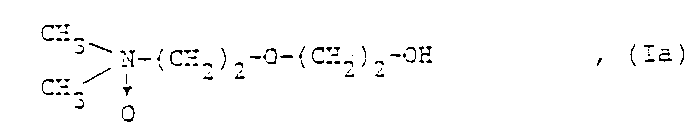

4. Verfahren zur Herstellung der in den Ansprüchen 1 bis 3 genannten Aminoxide durch Oxydation eines tertiären Amins, insbesondere durch Behandeln mit H₂O₂, dadurch gekennzeichnet, daß als tertiäres Amin eine Verbindung der allgemeinen Formel 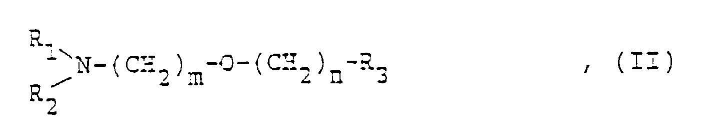 worin R₁, R₂, R₃, m und n obige Bedeutung haben, eingesetzt wird.

5. Form- bzw. spinnbare Lösung enthaltend Cellulose und ein tertiäres Aminoxid, dadurch gekennzeichnet, daß als tertiäres Aminoxid eine Verbindung der im Anspruch 1 genannten allgemeinen Formel (I), worin R₁, R₂, R₃, n und m obige Bedeutung haben, insbesondere ein tertiäres Aminoxid der im Anspruch 3 genannten Formel (Ia), vorgesehen ist und daß gegebenenfalls ein Hilfsstoff wie Wasser oder ein Nichtlösungsmittel enthalten ist.

6. Lösung nach Anspruch 5, dadurch gekennzeichnet, daß sie wasserfrei ist und über 10 Masse-% Cellulose enthält.

7. Lösung nach einem der Ansprüche 5 oder 6, dadurch gekennzeichnet, daß sie noch zusätzlich einen Stabilisator enthält.

8. Lösung nach Anspruch 7, dadurch gekennzeichnet, daß als Stabilisator eine Verbindung der Flavongruppe, vorzugsweise Rutin (3,3',4',5,7-Pentahydroxyflavon-3-rutinocid), Quercetin (3',3,3',5,7-Pentahydroxyflavon), oder Morin (2',3,4',5,7-Pentahydroxyflavon), insbesondere in Mengen von 0,001 bis 1,5 Masse-%, bezogen auf die Form- bzw. Spinnmasse vorgesehen ist.

9. Lösung nach Anspruch 7, dadurch gekennzeichnet, daß als Stabilisator ein Gemisch von H₂O₂ und Oxalsäure vorgesehen ist.

10. Lösung nach einem der Ansprüche 5 bis 9, dadurch gekennzeichnet, daß sie neben Cellulose noch ein weiteres Polymer enthält.

11. Lösung nach einem der Ansprüche 5 bis 10, dadurch gekennzeichnet, daß N-Methylmorpholin-N-oxid als weiteres Aminoxid vorgesehen ist.

12. Verfahren zur Herstellung einer form- bzw. spinnbaren Lösung gemäß einem oder mehreren der Ansprüche 5 bis 11, wobei eine Mischung aus cellulosehältigem Material und einem tertiären Aminoxid erwärmt wird, dadurch gekennzeichnet, daß der Mischung gegebenenfalls ein weiteres lösliches Polymer zugegeben wird und daß als tertiäres Aminoxid ein in den Ansprüchen 1, 2, 3 oder 5 genanntes Aminoxid, gegebenenfalls in Kombination mit N-Methylmorpholin-N-oxid, eingesetzt und eventuell vorhandenes Wasser gegebenenfalls abgezogen wird.

13. Verfahren zur Herstellung eines cellulosischen Formkörpers, wobei eine cellulosische Aminoxidlösung durch eine formgebende Vorrichtung gepreßt und gegebenenfalls verstreckt und koaguliert wird, dadurch gekennzeichnet, daß als cellulosische Aminoxidlösung eine Lösung gemäß einem der Ansprüche 5 bis 11 eingesetzt wird.

## Claims

1. Amine oxides of the general formula 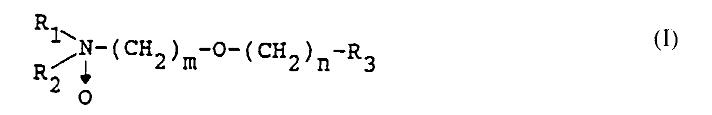 wherein R₁ and R₂ are alkyl groups having 1 to 4 C atoms, R₃ is hydrogen or a hydroxyl group, m is 2 or 3 and n is 1 or 2.

2. Amine oxides of the general formula (I) according to claim 1, wherein m is the number 2 and n is one of the numbers 1 or 2.

3. 2-(N,N-dimethylaminoethoxy)-ethanol-N-oxide of the formula

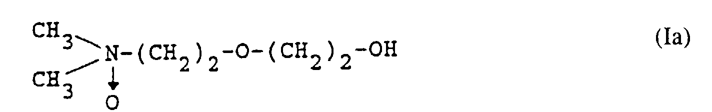

4. A process for preparing the amine oxides defined in claims 1 to 3 by oxidization of a tertiary amine, in particular by treatment with H₂O₂, characterized in that a compound of the general formula 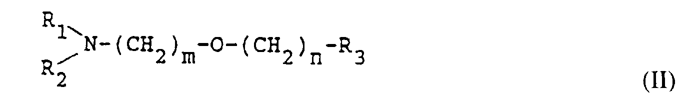 wherein R₁, R₂, R₃, m and n have the above meanings, is used as said tertiary amine.

5. A moldable or spinnable solution containing cellulose and a tertiary amine oxide, characterized in that a compound of the general formula (I) indicated in claim 1, wherein R₁, R₂, R₃, n and m have the above meanings, in particular a tertiary amine oxide of the formula (Ia) indicated in claim 3, is provided as said tertiary amine oxide and that optionally an auxiliary agent, such as water or a non-solvent is present.

6. A solution according to claim 5, characterized in that it is free of water and contains more than 10 % by mass of cellulose.

7. A solution according to any one of claims 5 or 6, characterized in that it additionally contains a stabilizer.

8. A solution according to claim 7, characterized in that a compound of the flavone group, preferably rutin (3,3',4',5,7-pentahydroxy-flavone-3-rutinocide), quercetine (3',3,3',5,7-pentahydroxy-flavone) or morin (2',3,4',5,7-pentahydroxy-flavone) is present as said stabilizer, in particular in amounts of from 0.001 to 1.5 % by mass, based on the molding or spinning mass.

9. A solution according to claim 7, characterized in that a mixture of H₂O₂ and oxalic acid is provided as said stabilizer.

10. A solution according to any one of claims 5 to 9, characterized in that it contains a further polymer in addition to cellulose.

11. A solution according to any one of claims 5 to 10, characterized in that N-methyl-morpholine-N-oxide is present as said additional amine oxide.

12. A process for preparing a moldable or spinnable solution according to one or several of claims 5 to 11, wherein a mixture of a cellulose-containing material and a tertiary amine oxide is heated, characterized in that a further soluble polymer is optionally added to said mixture and that an amine oxide defined in claim 1, 2, 3 or 5 is used as said tertiary amine oxide, optionally in combination with N-methylmorpholine-N-oxide, and that possibly present water is optionally drawn off.

13. A process for producing a cellulose molding by pressing a cellulose amine-oxide solution through a molding device and optionally drawing and coagulating the same, characterized in that a solution according to any one of claims 5 to 11 is used as said cellulose amine-oxide solution.

## Revendications

1. Oxydes d'amine de la formule générale 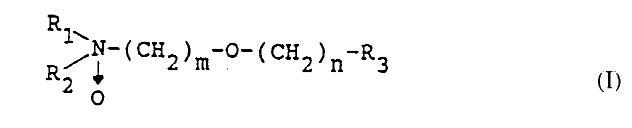 dans laquelle R₁ and R₂ représentent des groupes alkoyl ayant 1 à 4 atomes de carbone, R₃ est hydrogène ou un groupe hydroxyle, m signifie 2 ou 3 et n signifie 1 ou 2.

2. Oxydes d'amine de la formule générale (I) selon la revendication 1, dans laquelle m signifie le nombre 2 et n signifie un des nombres 1 ou 2.

3. 2-(N,N-dimethylaminoéthoxy)-éthanol-N-oxyde de la formule

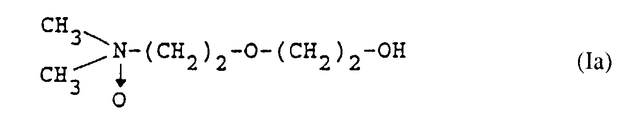

4. Procédé pour la préparation des oxydes d'amine définis dans les revendications 1 à 3 par oxydation d'une amine tertiaire, en particulier par traitement avec H₂O₂, caractérisé en ce qu'un composé de la formule générale 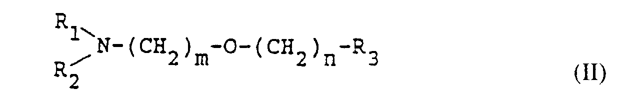 dans laquelle R₁, R₂, R₃, m and n sont tels que définis plus haut, est utilisé en tant qu'amine tertiaire.

5. Solution moulable ou filable contenant de la cellulose et un oxyde d'amine tertiaire, caractérisée en ce qu'un composé de la formule générale (I) définie dans la revendiation 1, dans laquelle R₁, R₂, R₃, n and m sont tels que définis plus haut, en particulier un oxyde d'amine tertiaire de la formule (Ia) définie dans la revendication 3, est pourvu en tant qu'oxyde d'amine tertiaire et en ce que, le cas échéant, un agent auxiliaire, comme de l'eau ou un non-solvent est renfermé.

6. Solution selon la revendication 5, caractérisée en ce qu'elle est dépourvue de l'eau et renferme plus de 10 % en masse de cellulose.

7. Solution selon l'une quelconque des revendications 5 ou 6, caractérisée en ce qu'elle contient en plus un stabilisateur.

8. Solution selon la revendication 7, caractérisée en ce qu'un composé du groupe flavone, de préférence, rutine (3,3',4',5,7-pentahydroxyflavone-3-rutinocide), quercétine (3',3,3',5,7-pentahydroxyflavone) ou morine (2',3,4',5,7-pentahydroxyflavone) est prévu en tant que stabilisateur, en particulier dans des quantités allant de 0,001 à 1,5 % en masse par rapport à la solution de moulage ou filage.

9. Solution selon la revendication 7, caractérisée en ce qu'un mélange de H₂O₂ et d'acide oxalique est prévu en tant que stabilisateur.

10. Solution selon l'une des revendications 5 à 9, caractérisée en ce qu'elle contient un polymère additionel outre de la cellulose.

11. Solution selon l'une des revendications 5 à 10, caractérisée en ce que N-méthyl-morpholine-N-oxyde est prévu en tant qu'oxyde d'amine additionel.

12. Procédé pour la préparation d'une solution moulable ou filable selon l'une ou plusieurs des revendications 5 à 11, dans lequel un mélange d'un matériau contenant de la cellulose et d'un oxyde d'amine tertiaire est chauffé, caractérisé en ce que, le cas échéant, un polymère soluble additionel est ajouté audit mélange et en ce qu'un oxyde d'amine défini dans l'une quelconque des revendications 1, 2, 3 ou 5, le cas échéant en association avec N-méthylmorpholine-N-oxyde, est utilisé en tant qu'oxyde d'amine tertiaire et, le cas échéant, de l'eau éventuellement présente est éliminée.

13. Procédé pour la production d'un corps moulé cellulosique, dans lequel une solution d'oxyde d'amine cellulosique est pressée à travers un dispositif moulant et, le cas échéant, est étirée et coagulée, caractérisé en ce qu'une solution selon l'une quelconque des revendications 5 à 11 est utilisée en tant que solution d'oxyde d'amine cellulosique.
